(19) Europäisches Patentamt
European Patent Office
Office européen des brevets

(11) Numéro de publication: **0 233 459 A1**

(12) # DEMANDE DE BREVET EUROPEEN

(21) Numéro de dépôt: **87100214.3**

(51) Int. Cl.[4]: **C07C 69/86**, C07C 67/00

(22) Date de dépôt: **09.01.87**

(30) Priorité: **22.01.86 FR 8600983**

(43) Date de publication de la demande: **26.08.87 Bulletin 87/35**

(84) Etats contractants désignés: **AT BE CH DE ES FR GB GR IT LI LU NL SE**

(71) Demandeur: **ARTIKA INTERNATIONAL (HONG-KONG) LIMITED**
**1101, Dominion Centre 43, Queen's Road East**
**HONG-KONG(HK)**

(72) Inventeur: **Voisin, Philippe**
**ZI Croix d'Hins**
**F-33380 Marcheprime(FR)**

(74) Mandataire: **Micheli, Michel-Pierre et al**
**MICHELI & CIE 118, Rue du Rhône Case Postale 47**
**CH-1211 Genève 6(CH)**

(54) **Médicament contenant un complexe acétylsalicylate de magnésium-urée et procédé pour la fabrication de ce complexe.**

(57) L'invention concerne un nouveau médicament à base de complexe acétylsalicylate de magnésium-urée, qui présente des propriétés antalgiques notamment.

Le complexe utilisé dans ce médicament est obtenu par réaction simultanée de l'acide acétylsalicylique et de carbonate de magnésium en présence d'urée.

EP 0 233 459 A1

**Médicament contenant un complexe acétylsalicylate de magnésium-urée et procédé pour la fabrication de ce complexe**

La présente invention concerne le domaine des médicaments à usage humain et vétérinaire, plus précisément celui des antalgiques, et se rapporte à un nouveau médicament contenant un complexe acétylsalicylate de magnésium-urée et à un procédé pour la fabrication de ce complexe.

L'aspirine est connue et utilisée depuis de très nombreuses années pour ses propriétés analgésique, antalgique et antipyrétique. Cependant, ce médicament présente, à côté d'une efficacité confirmée et dont la démonstration n'est plus à faire, quelques inconvénients dont le principal est sa très faible solubilité dans l'eau et son caractère acide.

On a donc recherché des moyens de remédier à ces inconvénients d'abord par la formation des sels avec des cations monovalents ou divalents, le caractère acide de l'aspirine étant ainsi supprimé et la solubilité du produit grandement améliorée.

Cependant, on obtenait ainsi des composés nettement instables, le radical acétyle de la molécule d'aspirine pouvant être hydrolysé, et les produits de dégradation étant l'acide acétique et l'acide salicylique, tous deux plus ou moins salifiés par le cation utilisé.

L'objet de la présente invention concerne un médicament contenant à titre de principe actif une quantité pharmaceutiquement acceptable de complexe acétylsalicylate de magnésium-urée (désigné ci-après par "ASMU"), et qui permet de remédier aux inconvénients des composés déjà connus.

Le procédé de préparation de l'ASMU, également objet de l'invention, fait appel à la réaction classique d'un acide sur un carbonate qu'il est capable de déplacer. Cependant l'originalité de ce procédé consiste à introduire dans le milieu réactionnel, en préalable à cette réaction, laquantité nécessaire d'urée defaçon à obtenir la formation du complexe au fur et à mesure de la formation du sel de magnésium suivant l'équation suivante:

$CO(NH_2) + 2C_8H_7O_2\text{-}COOH + CO_3Mg \rightarrow C_{19}H_{18}O_9N_2Mg + 2CO_2 + 2OH_2$

Le milieu réactionnel peut être constitué par de l'éthanol, de l'acétone ou de l'eau, mais pour des raisons économiques on préfère utiliser ce dernier solvant. La température à laquelle on opère est la température ambiante.

A titre d'exemple non limitatif, le protocole opératoire est le suivant.

Dans un récipient d'au moins deux litres de capacité on introduit 500 ml d'eau purifiée, dans laquelle on dissout 60 g d'urée. Puis 180,l g d'aspirine pulvérisée sont ajoutés au mélange et celui-ci est agité pendant cinq minutes jusqu'à obtention d'une suspension plus ou moins homogène. On ajoute alors, toujours sous agitation,80,3 g de carbonate de magnésium, ou bien plus facilement 97 g de carbonate basique de magnésium $(CO_3Mg)_4 \bullet Mg(OH)_2 \bullet 5H_2O$. L'addition de ce dernier composant doit être réalisée progressivement. La réaction chimique est complète lorsque tout le gaz carbonique s'est dégagé, soit après environ dix à quinze minutes. On obtient ainsi une solution dont la concentration en acétylsalicylate de magnésium-urée est voisine de 45 pour cent (p/v).

Le rendement est quantitatif, soit 275 g de produit environ.

Une fois la solution finale obtenue, clarifiée par filtration, il est procédé à une évaporation du solvant, sous vide, de façon à provoquer une cristallisation de la substance sous la forme d'une poudre microcristalline. Le produit obtenue ASMU présente les caractéristiques suivantes:

Formule brute : $C_{19}H_{18}O_9N_2Mg$

Poids moléculaire : 442,65

La formule développée est la suivante:

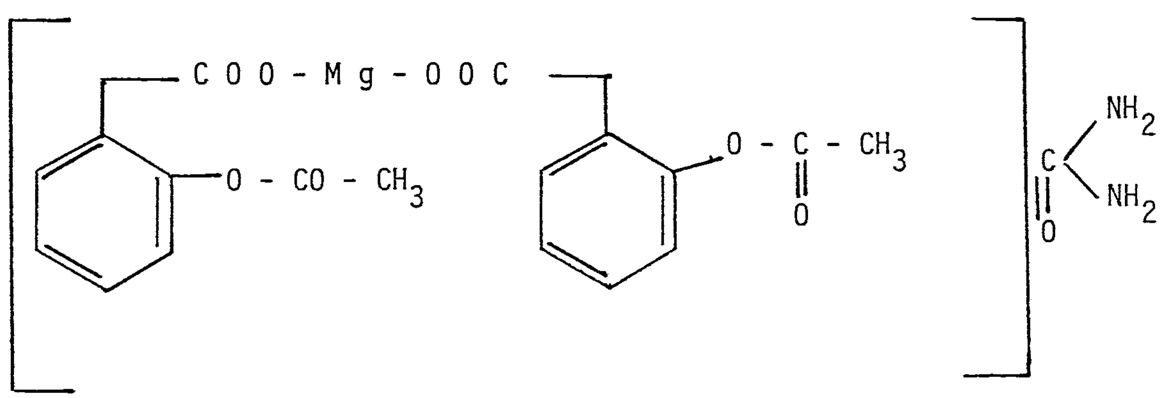

Ce produit ASMU est une poudre microcristalline blanche, à odeur et saveur rappelant celle de l'aspirine, très soluble dans l'eau, mais faiblement soluble dans l'alcool, l'acétone, et pratiquement insoluble dans les solvants chlorés et les hydrocarbures benzèniques et cyclaniques.

La solution aqueuse du complexe ASMU, à concentration de dix pour cent (p/v), présente une valeur de pH très proche de 6, donc proche de la neutralité et tout à fait compatible avec celui du pH sanguin.

La stabilité du complexe ASMU est bonne à l'état sec, et des essais de conservation dans cet état ont été menés pendant plus de deux ans et demi, sans qu'on ait pu observer d'altération notable.

Des études pharmacologiques réalisées sur ce complexe ASMU ont été pratiquées comparativement à celles de l'aspirine. Elles ont permis de mettre en évidence un comportement très différent de celui de cette dernière. En effet, si les activités générales de l'aspirine se retrouvent dans l'ASMU, elles en diffèrent de façon très considérable tant par le niveau beaucoup plus élevé de ces activités que par leur durée qui est fortement allongée.

A titre d'exemple, une présentation galénique possible de ce médicament est un sachet en complexe aluminium-polyéthylène-papier thermo-soudé, dans lequel le mélange de poudre, dont la composition est donnée ci-dessous, est réparti en doses unitaires de 0,70 g.

Composition centésimale de la poudre :

Acétylsalicylate de magnésium-urée (ASMU) 85,72 g

Arôme naturel de citron sur support lactose 14,28 g

BIODISPONIBILITE

On peut avoir une idée de la biodisponibilité d'un médicament en dosant la concentration de la substance dans le sang, c'est-à-dire au contact des tissus récepteurs. On a pu déterminer le volume des liquides extra-cellulaires chez l'Homme (0,15 à 0,19 litres par kg environ). La concentration maximum de la substance dans le plasma sanguin permet donc de mesurer le "rendement" obtenu entre l'administration d'une certaine dose et sa concentration dans l'organisme, par rapport à la concentration théorique qu'on devrait observer.

Cette première mesure doit ensuite être interprétée, parce qu'une substance médicamenteuse est éliminée plus ou moins rapidement après qu'elle a été introduite dans l'organisme vivant, du fait qu'elle peut subir des transformations plus ou moins rapides qui la rendent inactive ou moins active, et parce qu'elle a plus ou moins de facilité à pénétrer sous sa forme active dans les tissus vivants. On détermine donc une cinétique qui tient compte de ces divers paramètres, dans la mesure où on connaît leur existence et leur évolution.

La biodisponibilité de l'aspirine a été relativement bien étudiée, et on sait qu'une dose d'aspirine n'est que pour les deux tiers absorbée par l'organisme. De plus l'aspirine subit une transformation rapide dans la circulation sanguine, laquelle donne naisance à un métabolite, l'acide salicylique, par désacétylation, de sorte qu'on ne trouve plus guère que 10 pour 100 de la dose initiale sous la forme acétylée une heure après l'administration. Ces divers chiffres laissaient supposer que les effets observés lors de l'administration d'aspirine n'étaient pas ceux provoqués par cette dernière, mais par l'acide salicylique.

Cependant des travaux plus récents ont pu démontrer que les activités de l'aspirine étaient nettement plus intenses que celles de l'acide salicylique, que, par suite de divers processus (liaison avec les protéines plasmatiques, plus grande rapidité de passage à l'intérieur des tissus vivants, affinité particulière pour certains tissus développant une plus forte acidité, ce qui est le cas pour les tissus enflammés ,.. etc.), elle se trouvait en concentration suffisante à un moment donné de la cinétique, et que, enfin, son activité dans certains domaines persistant un certain temps après qu'elle ait disparu, on avait eu tendance à attribuer à l'acide salicylique des effets plus marqués au détriment de ceux de l'aspirine.

A la lumière des travaux qui ont été réalisés, on a pu déterminer que la cinétique de l'ASMU était très différente. Voici les points de différence, et les explications qui ont pu être formulées.

1) L'absorption de l'ASMU est pratiquement complète et plus rapide que celle de l'aspirine au niveau de la muqueuse gastrique. L'explication de ce comportement réside dans le fait que l'ASMU est soluble dans l'eau et que cette molécule est un complexe. Elle ne se trouve donc pas sous forme ionisée. Or, on sait que les substances chimiques ne traversent facilement les membranes cellulaires que si elles ne sont pas ionisées.

2) La désacétylation de l'aspirine ne peut s'opérer de la même mainère avec l'ASMU, toujours par suite de la forme complexe de la molécule. La fonction acétyle se trouve ainsi "dissimulée" à l'action de l'estérase responsable de la désacétylation de l'aspirine. C'est ainsi que l'acide salicylique ne représente

que 8,7 pour cent de l'ASMU circulant après une heure et quatorze pour cent après trois heures. Au cours des cinq heures suivant l'administration, la salicylémie moyenne se maintient autour de 10 pour cent de la quantité circulante. Ainsi, l'acétylsalicylémie provoquée par l'ASMU au bout d'une heure se trouvera entre dix et quinze fois plus élevée que celle produite par une dose équivalente d'aspirine.

3) La forme complexe de l'ASMU, c'est-à-dire non ionisée, favorise son passage à l'intérieur des tissus.

4) Enfin, la persistance de la forme acétylée de l'ASMU à des concentrations plasmatiques élevées explique la durée beau coup plus grande de l'activité. Après une administration orale de 0,8 g d'ASMU à un adulte, l'acétylsalicylémie représente après cinq heures 40 mg par litre de plasma, soit approximativement la moitié du chiffre initial.

Bien qu'appartenant à la même famille chimique que l'aspirine, l'ASMU voit son comportement biologique se différencier très considérablement de cette dernière, d'où des différences pharmacologiques en découlent.

L'étude de la biodisponibilité de l'ASMU a été effectuée sur diverses espèces animales et sur l'homme, en réalisant des dosages de la forme acétylée et désacétylée dans le temps. Les tableaux ci-dessous résument les résultats obtenus dans le plasma sanguin de l'homme (Tableau I), ainsi qu'au cours de l'élimination urinaire (Tableau II).

TABLEAU I - ASMU

Acétylsalicylémie et salicylémie chez l'homme après absorption orale de 0,0123 g/kg d'ASMU

| TEMPS | AAS ACIDE SALICYLIQUE (mg/l) | FORME ACETYLEE (mg/l) | % ACETYLE TOTAL |
|---|---|---|---|
| 1 | 0,9 | 24 | 96 |
| 2 | 0,8 | 24,8 | 96,5 |
| 3 | 0,6 | 24,4 | 97 |
| 4 | 1,6 | 16,1 | 90 |
| 5 | 0,4 | 12,7 | 89 |
| 6 | 0,1 | 8,4 | 85 |
| 7 | 0 | 0,3 | - |

Rappelons que pour une même dose d'aspirine administrée les quantités d'aspirine retrouvées dans le sang sont de l'ordre de 3 mg par litre, une heure après administration, accompagnées de 50 mg par litre d'acide salicylique.

TABLEAU II

| TEMPS | VOLUME MOYEN ml urine | AS TOTAL mg/ml | Q exprimée en mg ASMU | QUANTITE CUMULEE |
|---|---|---|---|---|
| 1 | 16 | 3,34 | 53 | 53 |
| 2 | 41 | 3,31 | 135 | 188 |
| 3 | 26 | 3,15 | 71 | 259 |
| 4 | 63 | 1,13 | 71 | 330 |
| 6 | 33 | 2,36 | 78 | 596 |
| 8 | 40 | 1,60 | 64 | 660 |
| 9 | 61 | 1,47 | 90 | 750 |
| 10 | 48 | 1,23 | 59 | 809 |
| 11 | 42 | 0,8 | 33 | 842 |

La dose administrée se trouve ainsi éliminée pour 98,5 pour 100 après onze heures.

ACTIVITE ANTI-INFLAMMATOIRE

L'inflammation est une suite de réactions des tissus vivants à une action déclenchante. Cette action peut être de nature traumatique ou bien chimique (substances irritantes) ou bien encore infectieuse. La réaction infectieuse peut être localisée ou généralisée. Elle met en jeu plusieurs mécanismes d'une importance relative variable suivant la nature de la cause. Les manifestations externes de l'inflammation comportent de la rougeur, un gonflement progressif des tissus lésés, accompagnées par une hyperthermie, des phénomènes douloureux et une hypersensibilité de la région atteinte.

En considérant cette manifestation sous l'angle de la physiologie, on note tout d'abord une adhérence des leucocytes et des plaquettes aux parois des vaisseaux capillaires. La perméabilité du réseau capillaire augmente, engendrant un oedème dans les tissus environnants. L'ensemble de ces manifestations est sous la dépendance de substances appelées médiateurs et qui sont libérées ou activées à la suite du stimulus inflammatoire.

On a ainsi invoqué l'intervention de l'histamine, qui est en effet un des agents de certaines inflammations. On a aussi mis en cause la libération de sérotonine. Mais son intervention semble en fait assez limitée. On a ensuite découvert le rôle impor tant de la bradykinine et du groupe des kinines. Ces diverses substances prennent naissance à la suite de réactions enzymatiques et ont sous leur dépendance au moins certaines phases du processus inflammatoire, ainsi que de la douleur. Enfin, on a pu mettre en évidence le rôle et la prépondérance d'un autre groupe de médiateurs, les prostaglandines,

Leurs actions ont été d'autant plus difficiles à étudier et à démontrer qu'elles prennent naissance presque toujours accompagnées d'un autre groupe de prostaglandines ayant des activités antagonistes des premières. Et ce qu'on peut observer est en général une modification de l'équilibre entre l'action de deux groupes de prostaglandines.

Cependent les connaissances concernant l'activité de cette famille de médiateurs se sont progressivement enrichies. On sait que le précurseur des prostaglandines est l'acide arachidonique, que sa transformation en prostaglandines nécessite l'intervention de cyclooxygénases (lesquelles sont dans certains cas un groupe d'enzymes dont une isomérase et une oxygénase). On sait aussi que l'aspirine intervient dans cette chaîne de transformation par inactivation des cyclo-oxygénases à la suite de leur acétylation. Ainsi, la synthèse de certaines prostaglandines induites par les processus de l'inflammation se trouve stoppée.

C'est ainsi que la deuxième vague de l'agrégation plaquettaire, qui est le début de la formation du thrombus (caillot) intravasculaire, et qui nécessite l'intervention des prostaglandines, est inhibée. C'est ainsi encore, qu'on a pu démontrer que la libération des kinines était sous la dépendance de l'intervention de certaines prostaglandines.

L'action inhibitrice de l'aspirine sur la formation de ces prostaglandines explique, au moins pour une part, son rôle anti-inflammatoire et antalgique.

On sait aussi que l'inflammation ne se résume pas à ces seuls phénomènes. D'autres voies existent ayant aussi pour précurseur l'acide arachidonique et les acides gras insaturés, et qui aboutissent à la formation de leukotriènes. Ces substances inter viennent elles aussi dans les processus inflammatoires et leur rôle n'est pas encore complètement élucidé. Cependant, on a pu démontrer que, dans cette autre voie de la formation de l'inflammation l'aspirine jouait aussi un rôle d'inhibiteur. Il convient cependant de noter que lors des expérimentations qui ont abouti à la connaissance actuelle des médicaments, telle que brièvement et schématiquement rapporté ci-dessus, les doses d'aspirine utilisables pour démontrer son activité étaient fréquemment dix fois supérieures aux doses thérapeutiques habituelles.

Au cours des études déjà réalisées sur l'ASMU, il a paru souhaitable également d'évaluer son activité anti-inflammatoire.

Deux tests de production d'oedème provoqué sur patte de rat, l'un au formol, et l'autre à l'ovalbumine ont été choisis. Dans les deux cas les mesures ont été opérées en incluant une série d'animaux traitée avec de l'aspirine, en comparaison de celle traitée avec l'ASMU. Les résultats ont montré une activité de l'ASMU en moyenne dix fois supérieure à celle de l'aspirine (résultats comparables dans les deux tests, et efficacité supérieure à trois heures qu'à une heure), comme cela ressort des tableaux III et IV ci-après:

TABLEAU III - Moyenne des volumes mesurés

| | TEMOINS | A S L | ASMU | AAS |
|---|---|---|---|---|
| $V_0$ $cm^3$ | 0,877±0,13 | 0,670±0,1 | 0,890±0,09 | 0,810±0,13 |
| $\Delta V_1$ heure | 0,676±0,022 | 0,490±0,04 | 0,150±0,04 | 0,580±0,03 |
| $\Delta V_3$ heures | 1,121±0,14 | 0,700±0,15 | 0,170±0,03 | 0,930±0,05 |
| | VARIATIONS DE VOLUME EXPRIME EN % | | | |
| A 1 heure | 77,1% | 50,16% | 16,48% | 72% |
| A 3 heures | 127,8% | 72,29% | 18,61% | 115,01% |

La dose d'anti-inflammatoire est de 20 mg/kg dans les trois cas, en équivalent aspirine.

TABLEAU IV - INDICE DE PROTECTION

| | A S L | ASMU | AAS |
|---|---|---|---|
| à 1 heure | 35% | 78,6% | 7% |
| à 3 heures | 43% | 85,4% | 10% |

ACTIVITE ANTALGIQUE

Cette activité consiste à atténuer ou à supprimer la sensation de douleur. Comme déjà mentionné lors de la brève étude sur l'inflammation, certains médiateurs provoquent la douleur, très vraisemblablement par action directe sur des terminaisons nerveuses sensorielles. Cependant cette activité antalgique est considérablement plus difficile à apprécier, d'autant que des composantes subjectives sont souvent mélées à celles qui sont objectives.

Cependant, quelques tests classiques ont été standardisés et ils fournissent des indications au moins comparatives.

Le test dit "de la plaque chauffante" a donc été pratiqué pour déterminer de la valeur de l'ASMU en comparaison avec l'aspirine. Le principe de la méthode consiste à poser une souris sur une plaque dont on a élevé la température en fonction du temps. On note la température à laquelle la souris saute, la sensation de chaleur devenant douleur. Cette température est relativement fixe pour chaque animal.

En recommançant la même expérience avec la souris après administration d'une dose d'un analgésique, on notera une température plus élevée qui sera fonction de la dose et de l'action antalgique de la substance.

Dans le tableau V ci-après ont été rassemblés les résultats, correspondants à une moyenne de dix essais obtenus pour l'aspirine (AAS), l'"ASPEGIC" (ASL) et l'ASMU.

Le dessin annexé représente la courbe de l'accroissement de la température tolérée en fonction du temps après administration, respectivement pour AAS, ASL et ASMU, selon le tableau V.

De ces résultats, on peut tirer la conclusion que l'"ASPEGIC" a une action plus importante que celle de l'aspirine, après une heure, mais plus courte, et que l'ASMU présente une action antalgique beaucoup plus prononcée et de durée beaucoup plus grande que celle des autres substances.

TABLEAU V - ETUDES DE L'ACTION ANTALGIQUE

| Δt(°C) / Temps en heures | AAS | ASL | ASMU |
|---|---|---|---|
| ½ | 0,90 | 1,25 | 3,70 |
| 1 | 1,15 | 2,15 | 4,75 |
| 2 | 1,40 | 1,90 | 4,90 |
| 3 | 0,75 | 0,45 | 4,90 |

ACTION ANTIPYRETIQUE

L'action antipyrétique d'une substance médicamenteuse consiste à ramener à sa valeur nominale la température interne d'un animal homéotherme, après qu'elle ait subi une élévation à la suite d'une infection, ou bien à la suite de substance pyrogènes, dont la source est, entre autres, dans les débris des bactéries tuées par stérilisation à la chaleur.

Le lapin est utilisé pour ce test, et aucune augmentation de la température n'a été constatée après administration de substances pyrogènes pour l'aspirine et l'ASMU, à une dose équivalente de 8,5 mg/kg.

EFFETS SECONDAIRES MANIFESTES PAR L'ASPIRINE ET L'ASMU

1) TOXICITE GENERALE

Des expérimentations ont été réalisées sur l'ASMU. La toxicité aiguë a été étudiée par la détermination classique de la $DL_{50}$, correspondant à la dose de substance qui, administrée à un groupe d'animaux, provoque la port de la moitié d'entre eux.

Les résultats ont montré que la toxicité aiguë de l'ASMU était diminuée d'environ 20 pour cent par rapport à l'équivalent aspirine sur la souris et le rat.

La toxicité chronique menée sur le rat et le porc, pendant une durée de quatre mois et à des doses cinq et dix fois supérieures aux doses thérapeutiques habituelles de l'aspirine, n'a pas provoqué d'anomalie particulière chez les animaux traités après les examens cliniques, et anatomopathologiques qui ont été pratiqués. En particulier aucun signe de lésion rénale ou stomacale n'a été décelé.

Il apparaît donc, que, par rapport aux résultats obtenus sur animaux, le complexe ASMU selon l'invention présente une toxicité générale qui est diminuée par rapport à celle de l'aspirine.

2) <u>ACTION IRRITANTE SUR LA PAROI GASTRIQUE</u>

En addition des résultats obtenus lors de l'étude de la toxicité générale de l'ASMU, une expérimentation particulière a été menée sur le rat pour déterminer de façon plus précise l'action irritant sur la muqueuse gastrique que peut développer l'ASMU par rapport à l'aspirine.

Le rat placé dans des conditions de contrainte (cage de petite dimension, n'interdisant pas le mouvement, mais empêchant l'animal de se déplacer et même de se retourner) pendant une durée de douze heures pendant plusieurs jours, développe rapidement une irritation de l'estomac qui évolue jusqu'à l'ulcère.

Sur cet animal sensibilisé, on peut tester, en même temps que la contrainte, l'action de médicaments irritants qui accélèrent le processus de formation de l'ulcère. En fin d'expérience, on sacrifie les animaux, et l'examen de la muqueuse gastrique permet de tirer des conclusions sur le pouvoir irritant des substances en comparaison avec les produits de référence.

Les résultats obtenus ont montré que l'ASMU provoquait une irritation comparable, mais légèrement supérieure à celle des témoins, alors que l'aspirine provoquait des ulcères sur 80 pour cent des animaux traités.

3) <u>ACTION SUR LA COAGULATION SANGUINE</u>

Une expérience a été menée sur lapins, afin de déterminer le temps de saignement modifié par l'administration d'ASMU et d'aspirine à doses équivalentes.

Ces mesures du temps de saignement ont donné les résultats suivants:

<u>TABLEAU VI</u> - ALLONGEMENT DU TEMPS DE SAIGNEMENT

(oreille de lapin)

| | |
|---|---|
| ASPIRINE | 66% |
| A S L (ASPEGIC) | 35% |
| A S M U | 7% |

Il est à noter que l'ASMU paraît avoir très peu d'influence sur le temps de saignement, et que cela peut paraître à priori contradictoire avec l'activité d'inhibition de la synthèse des prostaglandines.

Cependant, ce résultat est parfaitement cohérent avec l'activité générale plus intense de l'ASMU.

Comme exposé ci-dessus, l'aspirine inhibe la formation des prostaglandines, en empêchant par acétylation, l'action de cyclo-endopéroxydases, dont la fonction est de transformer l'acide arachidonique en diverses prostaglandines suivant les endopéroxydes synthétisés (fournissant ainsi les prostaglandines $G_2$, $A_2$, et le thromboxane $A_2$, à activité agrégante et vaso-constrictrice marquées),

Cependant, à côté de ce processus d'agrégation, il existe un processus parallèle prenant naissance à partir des mêmes endoperoxydes et qui fournit une substance appelée prostacyline par action d'une prostacycline synthétase. Or, l'activté de cette prostacycline est antagoniste des prostaglandines citées plus haut, c'est-à-dire désagrégante et vasodilatatrice. Ce processus est, lui aussi, inhibé par l'aspirine. Cependant, il est nécessaire d'utiliser de fortes doses, de l'ordre de cinq à dix fois supérieures aux doses thérapeutiques.

Dans ces conditions, on n'observe plus l'allongement du temps de saignement. Or, c'est l'effet que produit l'ASMU à doses équivalentes aux doses thérapeutiques de l'aspirine, soit 0,5 à 1 g par jour pour un adulte.

On peut enfin ajouter que le couple d'activité antagoniste prostaglandines-prostacycline n'est qu'un mécanisme régulateur de la coagulation du sang, cette dernière étant réalisée par d'autres mécanismes.

4) <u>ACTIVITE SUR L'AGREGATION PLAQUETTAIRE</u>

Comme mentionné plus haut, le phénomène d'agrégation plaquettaire est une des premières manifestations de l'installation d'un processus inflammatoire. A la suite de l'induction de ce processus, la paroi interne des capillaires atteints est lésée. Les plaquettes sanguines viennent adhérer à cette paroi, subissent une transformation, et d'autres plaquettes viennent adhérer à ces plaquettes transformées, réalisant un agrégat qui est ensuite consolidé par la formation d'un caillot de fibrine. On a ainsi décrit cette intervention des plaquettes comme étant constituée par une première vague de l'agrégation plaquettaire (l'adhésion initiale des plaquettes à la paroi vasculaire) suivie d'une seconde vague (la formation d'un agrégat).

L'action de l'aspirine réside dans l'inhibition de la deuxième vague de l'agrégation, par le blocage de la synthèse de prostaglandines spécifiques, lesquelles induisent la transformation des plaquettes adhérant à la paroi vasculaire.

Dans ces conditions, l'adhésion primaire des plaquettes est un processus réversible.

Certains troubles, certaines maladies et certaines prédispositions à des maladies sont provoqués par une trop grande aptitude à l'agrégation plaquettaire. Les attaques d'ischémie transitoire, les maladies des artères coronariennes, le purpura thrombocytopénique thrombotique, les embolies veineuses post-opératoires ont été traités par l'aspirine avec des résultats parfois favorables et parfois décevants.

L' ASMU devrait apporter un complément d'activité qui a parfois fait défaut à l'aspirine.

On a aussi mis en évidence des tendances à l'agrégation plaquettaire dans les maladies suivantes. Ainsi, un certain nombre de malades souffrent de troubles cérébro-vasculaires et montrent une agrégation plaquettaire spontanée. Les malades atteints par le diabète débutant ou confirmé présentent une deuxième vague de l'agrégation plaquettaire fortement augmentée, en corrélation avec des taux anormaux du glucose et des acides gras libres dans le sang. Des malades souffrant d'ischémie cardiaque transitoire ou de troubles des artères périphériques ont montré des anomalies de l'agrégation plaquettaire. Ces manifestations sont certainement liées à un déplacement de l'équilibre des prostaglandines. Des traitements avec l'aspirine ont été essayés et ont démontré son efficacité pour contrôler ces anomalies.

Cependant, dans certains cas, les doses nécessaires étaient très élevées, et il est à peu près certain que l'ASMU pourrait avantageusement être prescrit.

Enfin, toujours dans ce domaine de l'agrégation plaquettaire induite dans le réseau vasculaire, il faut noter que lors de la greffe d'organes ou lors de la mise en place de prothèses, cardiaque par exemple, une agrégation plaquettaire intempestive est parfois observée, qui compromet le travail chirurgical réalisé. l'ASMU trouvera aussi dans ce domaine une application sérieuse par l'action inhibitrice importante qu'il montre sur la synthèse des prostaglandines inductrices.

A titre d'exemple d'application thérapeutique inhabituelle, on a pu observer l'action curative de l'ASMU dans le traitement d'ostéochondrites déformantes infectieuses. Dans tous les cas traités, l'inflammation a regressé, puis disparu et l'articulation atteinte a recouvré sa mobilité. Seule la déformation a persisté lorsqu'elle existait, comme on pouvait s'y attendre.

## Revendications

1. Médicament contenant à titre de principe actif une quantité pharmaceutiquement acceptable du complexe acétylsalicylate de magnésium-urée représenté par la formule générale suivante:

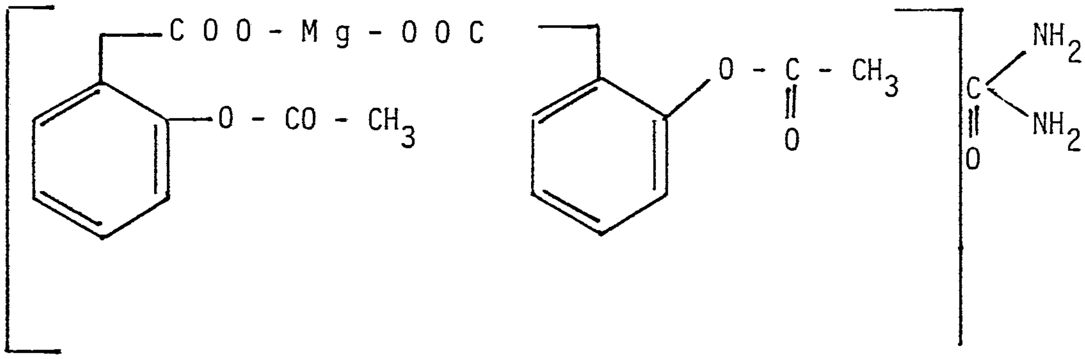

de formule brute $C_{19}H_{18}O_9N_2Mg$ et ayant une masse moléculaire de 442,65.

2. Médicament pour administration orale selon la revendication 1 sous la forme de doses unitaires d'environ 0,70g, contenant chacune environ 85% poids du complexe.

3. Médicament selon la revendication 2, caractérisé par le fait qu'il contient environ 15% en poids d'un arôme sur un support lactose.

4. Procédé pour la fabrication du complexe acétylsalicylate de magnésium-urée présent dans le médicament selon la revendication 1, caractérisé par le fait qu'on effectue la salification de l'acide acétylsalicylique avec du carbonate de magnésium ou du carbonate basique de magnésium $(CO_3Mg)_4 \bullet Mg(OH)_2 \bullet 5\ H_2O$, en présence d'une quantité d'urée nécessaire pour obtenir la formation du complexe au fur et à mesure de la formation du sel de magnésium.

5. Procédé selon la revendication 4, caractérisé par le fait que la réaction est effectuée à la température ambiante dans un solvant choisi parmi l'éthanol, l'acétone et l'eau.

Revendications pour les Etats contractants suivants: AT, GR, et ES

1. Procédé pour la fabrication d'un complexe acétylsalicylate de magnésium-urée représenté par la formule générale suivante:

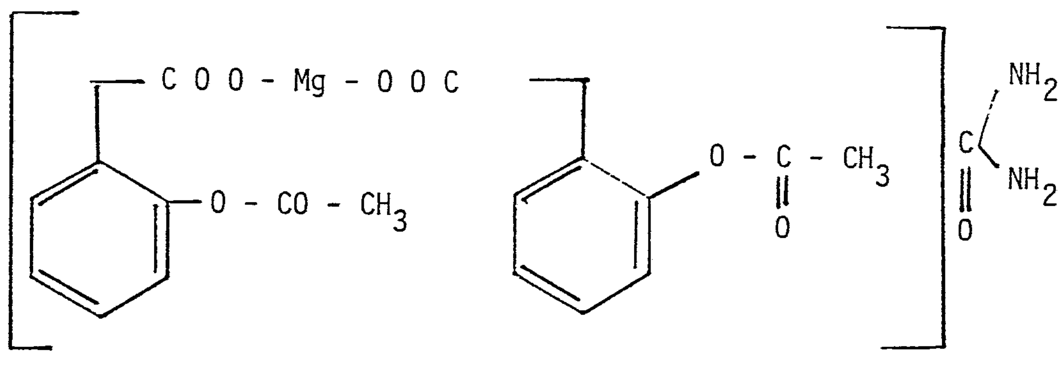

de formule brute $C_{19}H_{18}O_9N_2Mg$ et ayant une masse moléculaire de 442,65, caractérisé par le fait qu'on effectue la salification de l'acide acétylsalicylique avec du carbonate de magnésium en présence d'une quantité d'urée nécessaire pour obtenir la formation du complexe au fur et à mesure de la formation du sel de magnésium.

2. Procédé selon la revendication 1, caractérisé par le fait que la salification est effectuée avec du carbonate basique de magnésium, tel que le $(CO_3Mg)_4 \bullet Mg(OH)_2 \bullet 5\ H_2O$.

3. Procédé selon la revendication 1 ou la revendication 2, caractérisé par le fait que la réaction est effectuée à la température ambiante dans un solvant choisi parmi l'éthanol, l'acétone et l'eau.

4. Procédé pour la fabrication d'un médicament contenant à titre de principe actif le complexe acétylsalicylate de magnésium-urée obtenu par le procédé selon la revendication 1, caractérisé par le fait qu'on mélange une quantité pharmaceutiquement acceptable dudit complexe avec un support acceptable pharmaceutiquement.

5. Procédé selon la revendication 4, caractérisé par le fait qu'on mélange environ 85% en poids du complexe avec environ 15% d'un support lactose contenant un arôme sous forme de poudre.

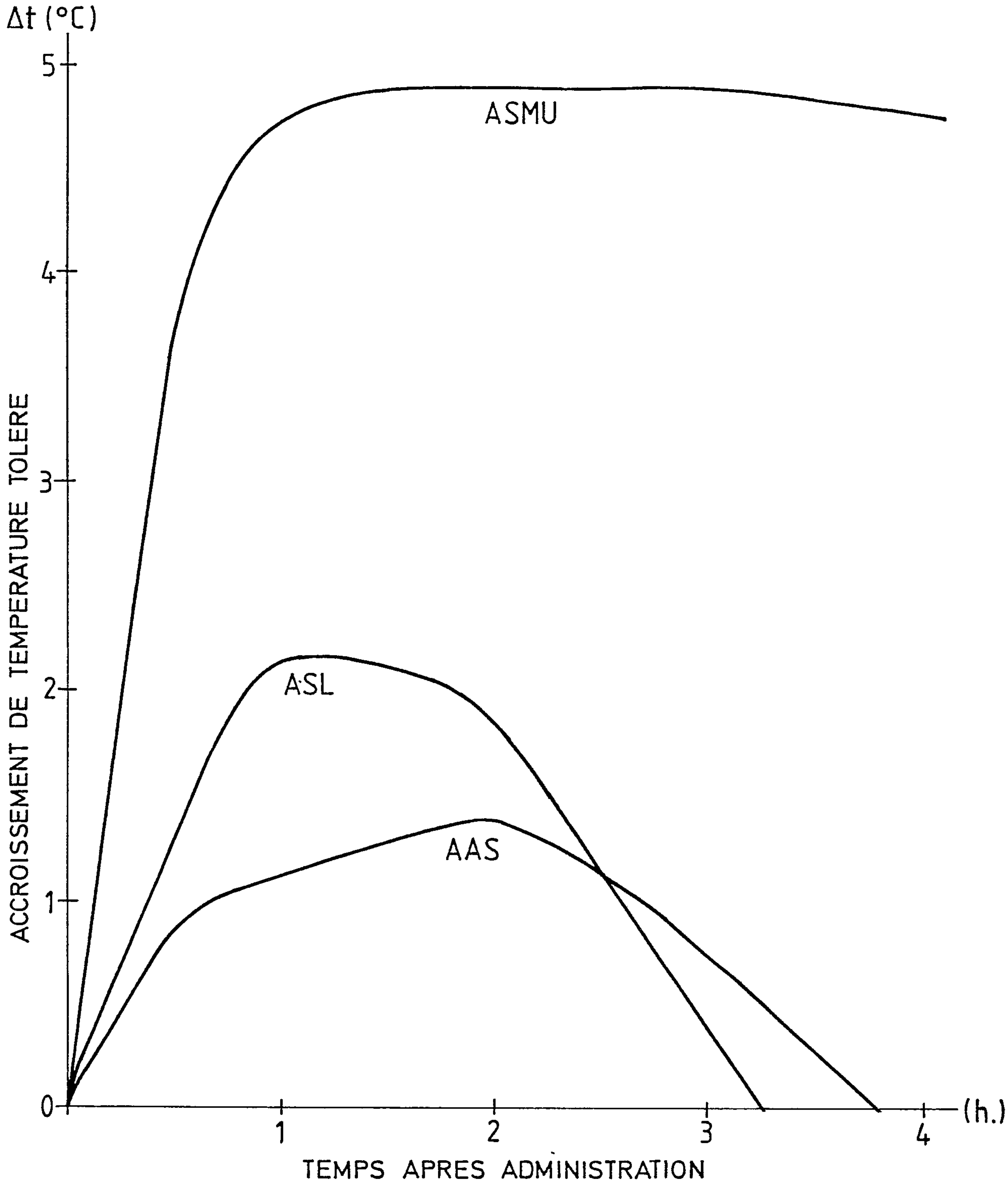
Δt (°C)
5
4
3
2
1
0
ASMU
ASL
AAS
ACCROISSEMENT DE TEMPERATURE TOLERE
1
2
3
4
(h.)
TEMPS APRES ADMINISTRATION

Office européen des brevets

# RAPPORT DE RECHERCHE EUROPEENNE

Numéro de la demande

EP 87 10 0214

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 492 368 (P. VOISIN)<br>* Page 1, lignes 22-30; page 2, lignes 20-38; page 3, revendications 1-3 * | 1-4 | C 07 C 69/86<br>C 07 C 67/00 |
| X | DE-A- 845 944 (A. SCHMIDGALL)<br>* Page 2, lignes 21-33 * | 1 | |

DOMAINES TECHNIQUES RECHERCHES (Int. Cl.4)

C 07 C 69/00
C 07 C 67/00

Le présent rapport de recherche a été établi pour toutes les revendications

| Lieu de la recherche | Date d'achèvement de la recherche | Examinateur |
|---|---|---|
| LA HAYE | 09-04-1987 | KINZINGER J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82